(19) Europäisches Patentamt / European Patent Office / Office européen des brevets

(11) EP 3 362 055 B1

(12) # EUROPEAN PATENT SPECIFICATION

(45) Date of publication and mention of the grant of the patent: **18.01.2023 Bulletin 2023/03**

(21) Application number: **16781135.5**

(22) Date of filing: **13.10.2016**

(51) International Patent Classification (IPC):
***A61K 31/00*** *(2006.01)* ***A61K 31/7048*** *(2006.01)*
***A61P 21/00*** *(2006.01)*

(52) Cooperative Patent Classification (CPC):
**A61K 31/7048; A61K 31/00; A61P 3/06; A61P 3/10; A61P 21/00; A61P 43/00**

(86) International application number:
**PCT/EP2016/074601**

(87) International publication number:
**WO 2017/064193 (20.04.2017 Gazette 2017/16)**

(54) **SGLT-2 INHIBITOR FOR USE IN THE TREATMENT OF A METABOLIC MYOPATHY**

SGLT-2-INHIBITOREN ZUR BEHANDLUNG EINER METABOLISCHEN MYOPATHIE

INHIBITEUR DE SGLT-2 DESTINÉ À ÊTRE UTILISÉ DANS LE TRAITEMENT D'UNE MYOPATHIE MÉTABOLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.10.2015 US 201562242030 P**
**09.06.2016 US 201662347747 P**

(43) Date of publication of application:
**22.08.2018 Bulletin 2018/34**

(73) Proprietor: **Boehringer Ingelheim International GmbH**
**55216 Ingelheim am Rhein (DE)**

(72) Inventor: **MAYOUX, Eric Williams**
**55216 Ingelheim Am Rhein (DE)**

(74) Representative: **Simon, Elke Anna Maria et al**
**Boehringer Ingelheim GmbH**
**Binger Strasse 173**
**55216 Ingelheim am Rhein (DE)**

(56) References cited:

| | |
|---|---|
| **WO-A1-2010/092123** | **WO-A1-2014/161918** |
| **WO-A1-2016/046150** | **WO-A2-2010/049678** |
| **CN-A- 101 638 423** | **US-A1- 2015 272 977** |

- **CETRONE MICHELA ET AL: "Effects of the antidiabetic drugs on the age-related atrophy and sarcopenia associated with diabetes type II.", CURRENT DIABETES REVIEWS 2014, vol. 10, no. 4, 2014, pages 231-237, XP002764922, ISSN: 1875-6417**
- **NISHIMURA ET AL.: CARDIOVASCULAR DIABETOLOGY, vol. 14, no. 11, 30 January 2015 (2015-01-30), pages 1-13,**
- **DANIELE ET AL.: DIABETES CARE, vol. 39, November 2016 (2016-11), pages 2036-2041,**
- **ZINMAN ET AL.: THE NEW ENGLAND JOURNAL OF MEDICINE, vol. 373, 2015, pages 2117-2128,**
- **B. Katirji: "Metabolic Myopathies", , 19 October 2016 (2016-10-19), Retrieved from the Internet: URL:https://emedicine medscape.com/article/1173338-print [retrieved on 2020-07-13]**
- **FREY ET AL.: BIOCHIMICA ET BIOPHYSICA ACTA, vol. 1863, no. 1, 2017, pages 284-291,**
- **BRANCO ET AL.: EUROPEAN JOURNAL OF CLINICAL INVESTIGATION, vol. 46, no. 3, 2016, pages 285-298,**
- **REASON ET AL.: JOURNAL OF RARE DISORDERS, vol. 3, 2017, pages 1-5,**
- **G.F. CAHILL ET AL.: TRANSACTIONS OF THE AMERICAN CLINICAL AND CLIMATOLOGICAL ASSOCIATION, vol. 114, 2003, pages 149-161,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SUZUKI YOSHIHIKO ET AL: "Gain of muscle strength in mitochondrial diabetes treated with SGLT2 inhibitors", DIABETES RESEARCH AND CLINICAL PRACTICE, vol. 120, 29 October 2016 (2016-10-29), ISSN: 0168-8227, DOI: 10.1016/S0168-8227(16)31105-6**

## Description

### Technical Field of the Invention

The invention relates to an SGLT2-inhibitor for use in a method for treating, delaying or slowing down the progression of a metabolic myopathy as described herein.

### Background of the Invention

Type 2 diabetes is an increasingly prevalent disease that due to a high frequency of complications leads to a significant reduction of life expectancy. Because of diabetesassociated microvascular complications, type 2 diabetes is currently the most frequent cause of adult-onset loss of vision, renal failure, and amputations in the industrialized world. In addition, the presence of type 2 diabetes is associated with a two to five fold increase in cardiovascular disease risk.

After long duration of disease, most patients with type 2 diabetes will eventually fail on oral therapy and become insulin dependent with the necessity for daily injections and multiple daily glucose measurements.

The UKPDS (United Kingdom Prospective Diabetes Study) demonstrated that intensive treatment with metformin, sulfonylureas or insulin resulted in only a limited improvement of glycemic control (difference in HbA1c ~0.9%). In addition, even in patients within the intensive treatment arm glycemic control deteriorated significantly over time and this was attributed to deterioration of β-cell function. Importantly, intensive treatment was not associated with a significant reduction in macrovascular complications, i.e. cardiovascular events. Therefore many patients with type 2 diabetes remain inadequately treated, partly because of limitations in long term efficacy, tolerability and dosing inconvenience of existing antihyperglycemic therapies.

Oral antidiabetic drugs conventionally used in therapy (such as e.g. first- or second-line, and/or mono- or (initial or add-on) combination therapy) include, without being restricted thereto, metformin, sulphonylureas, thiazolidinediones, glinides and α-glucosidase inhibitors.

The high incidence of therapeutic failure is a major contributor to the high rate of long-term hyperglycemia-associated complications or chronic damages (including micro- and macrovascular complications such as e.g. diabetic nephrophathy, retinopathy or neuropathy, or cardiovascular complications) in patients with type 2 diabetes.

Therefore, there is an unmet medical need for methods, medicaments and pharmaceutical compositions with a good efficacy with regard to glycemic control, with regard to diseasemodifying properties and with regard to reduction of cardiovascular morbidity and mortality while at the same time showing an improved safety profile.

SGLT2 inhibitors inhibitors represent a novel class of agents that are being developed for the treatment or improvement in glycemic control in patients with type 2 diabetes. Glucopyranosyl-substituted benzene derivative are described in the prior art as SGLT2 inhibitors, for example in WO 01/27128, WO 03/099836, WO 2005/092877, WO 2006/034489, WO 2006/064033, WO 2006/117359, WO 2006/117360, WO 2007/025943, WO 2007/028814, WO 2007/031548, WO 2007/093610, WO 2007/128749, WO 2008/049923, WO 2008/055870, WO 2008/055940. The glucopyranosyl-substituted benzene derivatives are proposed as inducers of urinary sugar excretion and as medicaments in the treatment of diabetes.

Renal filtration and reuptake of glucose contributes, among other mechanisms, to the steady state plasma glucose concentration and can therefore serve as an antidiabetic target. Reuptake of filtered glucose across epithelial cells of the kidney proceeds via sodium-dependent glucose cotransporters (SGLTs) located in the brush-border membranes in the tubuli along the sodium gradient. There are at least 3 SGLT isoforms that differ in their expression pattern as well as in their physico-chemical properties. SGLT2 is exclusively expressed in the kidney, whereas SGLT1 is expressed additionally in other tissues like intestine, colon, skeletal and cardiac muscle. SGLT3 has been found to be a glucose sensor in interstitial cells of the intestine without any transport function. Potentially, other related, but not yet characterized genes, may contribute further to renal glucose reuptake. Under normoglycemia, glucose is completely reabsorbed by SGLTs in the kidney, whereas the reuptake capacity of the kidney is saturated at glucose concentrations higher than 10mM, resulting in glucosuria ("diabetes mellitus"). This threshold concentration can be decreased by SGLT2-inhibition. It has been shown in experiments with the SGLT inhibitor phlorizin that SGLT-inhibition will partially inhibit the reuptake of glucose from the glomerular filtrate into the blood leading to a decrease in blood glucose concentrations and to glucosuria.

Metabolic myopathies refer to groups of heterogeneous muscle disorders caused by defective gene involved in energy production for muscles activities. Defects in any of these pathways-glycogen catabolism (glycogenolysis and glycolysis), fatty acid oxidation, Krebs cycle, or mitochondrial respiratory chain and oxidative phosphorylation affect predominantly muscle because of its high energy requirements, particularly during exercise. Because most of the enzyme defects are partial, many of these diseases manifest in adulthood with isolated muscle symptoms and similar clinical features. From a clinical point of view, metabolic myopathies can be categorized into two different groups: 1) those that show symptoms and signs related to exercise; exercise intolerance, cramps, myalgias, myoglobinuria, and 2) those with

fixed symptoms, such as muscle weakness, often associated with systemic involvement (for example endocrinopathies or encephalopathies). The quality of life of patients is significantly altered and they are limited in their activities, some in a wheelchair. A typical therapy is to avoid exercise, especially during fasting or during periods with superimposed infections. Treatments also include dietary modifications, depending on the metabolic pathway to produce ATP that is altered. The consumption of high carbohydrate diet or L-carnitine supplementation, before and during exercise, is recommended especially in patients suffering from disorder in the use of lipid for ATP production. Enzyme replacement remains an attractive treatment prospect but not yet available. Gene therapy potentially holds the key to developing a cure for metabolic muscle disease.

The document CN101638423 discloses phlorizin derivatives and a variety of different therapeutic indications including energy metabolism abnormal diseases, for example various myopathies.

Therefore there is an unmet medical need to improve tolerance to exercise and associated quality of life, in particular in patients with metabolic myopathies.

**Aim of the present invention**

One aim of the present invention is to provide a pharmaceutical composition for use in a method for improving the health of a subject.

A further aim of the present invention is to provide a pharmaceutical composition for use in a method for improving a marker of health in a subject.

A further aim of the present invention is to provide a pharmaceutical composition for use in a method to improve tolerance to exercise and associated quality of life, in particular in patients with metabolic myopathies.

Further aims of the present invention become apparent to the one skilled in the art by description hereinbefore and in the following and by the examples.

**Summary of the Invention**

In one aspect, present invention provides a SGLT-2 inhibitor for use in a method of treating, delaying or slowing down the progression of a metabolic myopathy in a patient, said method comprising administering to the patient the SGLT-2 inhibitor wherein the SGLT2 inhibitor is selected from a glucopyranosyl-substituted benzene derivative of the formula (I)

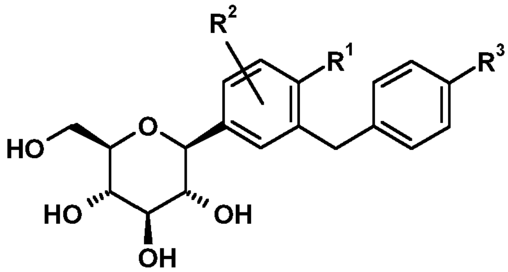

I

wherein $R^1$ denotes Cl or methyl; $R^2$ denotes H, methyl, methoxy or hydroxy and $R^3$ denotes ethyl, cyclopropyl, ethynyl, ethoxy, (*R*)-tetrahydrofuran-3-yloxy or (*S*)-tetrahydrofuran-3-yloxy.

In one aspect, the present invention provides the SGLT2 inhibitor as defined hereinbefore and hereinafter for use in a method for treating a metabolic myopathy in a patient, for example a metabolic myophathy from a glycogen or lipid metabolism disorder, comprising administering to the patient the SGLT-2 inhibitor. In one embodiment, the SGLT2 inhibitor for use in a method as defined hereinbefore and hereinafter replaces or mimicks a specific diet to increase energy production in organs, especially in muscles. In one embodiment, the SGLT2 inhibitor for use in a method as defined hereinbefore and hereinafter is in addition to a specific diet to increase energy production in organs, especially in muscles.

In one aspect, the present invention provides a SGLT2 inhibitor for use in a method as defined hereinbefore and hereinafter for delaying or slowing down the progression of a metabolic myopathy in a patient, for example a metabolic myophathy from a glycogen or lipid metabolism disorder, comprising administering to the patient a SGLT-2 inhibitor. In one embodiment, the SGLT2 inhibitor for use in a method as defined hereinbefore and hereinafter replaces or mimicks a specific diet to increase energy production in organs, especially in muscles. In one embodiment, the SGLT2 inhibitor for use in a method as defined hereinbefore and hereinafter is in addition to a specific diet to increase energy production in organs, especially in muscles.

In one aspect, a metabolic myopathy is a glycogen storage disease (GSD), a fatty acid oxidation defect (FAODs)

or a mitochondrial myopathy.

In one aspect, the SGLT2 inhibitor is administered as defined hereinbefore and hereinafter. In one aspect, the subject is a patient having type 2 diabetes mellitus.

In one aspect, the patient is a patient having type 2 diabetes mellitus.

In one aspect the SGLT-2 inhibitor is empagliflozin (compound (1.9)).

In one aspect the SGLT2 inhibitor for use in a method as defined hereinbefore and hereinafter further comprises a (non-claimed) method for

- preventing, slowing the progression of, delaying or treating a metabolic disorder selected from the group consisting of type 1 diabetes mellitus, type 2 diabetes mellitus, impaired glucose tolerance (IGT), impaired fasting blood glucose (IFG), hyperglycemia, postprandial hyperglycemia, overweight, obesity, metabolic syndrome and gestational diabetes; or
- improving glycemic control and/or for reducing of fasting plasma glucose, of postprandial plasma glucose and/or of glycosylated hemoglobin HbA1c; or
- preventing, slowing, delaying or reversing progression from impaired glucose tolerance (IGT), impaired fasting blood glucose (IFG), insulin resistance and/or from metabolic syndrome to type 2 diabetes mellitus; or
- preventing, slowing the progression of, delaying or treating of a condition or disorder selected from the group consisting of complications of diabetes mellitus such as cataracts and micro- and macrovascular diseases, such as nephropathy, retinopathy, neuropathy, tissue ischaemia, diabetic foot, arteriosclerosis, myocardial infarction, accute coronary syndrome, unstable angina pectoris, stable angina pectoris, stroke, peripheral arterial occlusive disease, cardiomyopathy, heart failure, heart rhythm disorders and vascular restenosis; or
- reducing body weight and/or body fat, or preventing an increase in body weight and/or body fat, or facilitating a reduction in body weight and/or body fat; or
- preventing, slowing, delaying or treating the degeneration of pancreatic beta cells and/or the decline of the functionality of pancreatic beta cells and/or for improving and/or restoring the functionality of pancreatic beta cells and/or restoring the functionality of pancreatic insulin secretion; or
- preventing, slowing, delaying or treating diseases or conditions attributed to an abnormal accumulation of ectopic fat; or
- maintaining and/or improving the insulin sensitivity and/or for treating or preventing hyperinsulinemia and/or insulin resistance;
- preventing, slowing progression of, delaying, or treating new onset diabetes after transplantation (NODAT) and/or post-transplant metabolic syndrome (PTMS);
- preventing, delaying, or reducing NODAT and/or PTMS associated complications including micro- and macrovascular diseases and events, graft rejection, infection, and death;
- treating hyperuricemia and hyperuricemia associated conditions;
- treating or prevention kidney stones;
- treating hyponatremia;

in a patient in need thereof characterized in that the SGLT2 inhibitor is administered, as defined hereinbefore and hereinafter.

In one aspect, the present invention provides the advantage of avoiding calorie restriction or intermittent fasting by the subject.

Further aspects of the present invention become apparent to the one skilled in the art by the description hereinbefore and in the following and by the examples.

## Definitions

The term **"active ingredient"** of a pharmaceutical composition according to the present invention means the SGLT2 inhibitor according to the present invention. An "active ingredient is also sometimes referred to herein as an "active substance".

The term **"body mass index"** or **"BMI"** of a human patient is defined as the weight in kilograms divided by the square of the height in meters, such that BMI has units of $kg/m^2$.

The term **"overweight"** is defined as the condition wherein the individual has a BMI greater than or 25 $kg/m^2$ and less than 30 $kg/m^2$. The terms "overweight" and "pre-obese" are used interchangeably.

The term **"obesity"** is defined as the condition wherein the individual has a BMI equal to or greater than 30 $kg/m^2$. According to a WHO definition the term obesity may be categorized as follows: the term "class I obesity" is the condition wherein the BMI is equal to or greater than 30 $kg/m^2$ but lower than 35 $kg/m^2$; the term "class II obesity" is the condition wherein the BMI is equal to or greater than 35 $kg/m^2$ but lower than 40 $kg/m^2$; the term "class III obesity" is

the condition wherein the BMI is equal to or greater than 40 $kg/m^2$.

The term **"visceral obesity"** is defined as the condition wherein a waist-to-hip ratio of greater than or equal to 1.0 in men and 0.8 in women is measured. It defines the risk for insulin resistance and the development of pre-diabetes.

The term **"abdominal obesity"** is usually defined as the condition wherein the waist circumference is > 40 inches or 102 cm in men, and is > 35 inches or 94 cm in women. With regard to a Japanese ethnicity or Japanese patients abdominal obesity may be defined as waist circumference ≥ 85 cm in men and ≥ 90 cm in women (see e.g. investigating committee for the diagnosis of metabolic syndrome in Japan).

The term **"euglycemia"** is defined as the condition in which a subject has a fasting blood glucose concentration within the normal range, greater than 70 mg/dL (3.89 mmol/L) and less than 100 mg/dL (5.6 mmol/L). The word "fasting" has the usual meaning as a medical term.

The term **"hyperglycemia"** is defined as the condition in which a subject has a fasting blood glucose concentration above the normal range, greater than 100 mg/dL (5.6 mmol/L). The word "fasting" has the usual meaning as a medical term.

The term **"hypoglycemia"** is defined as the condition in which a subject has a blood glucose concentration below the normal range, in particular below 70 mg/dL (3.89 mmol/L).

The term **"postprandial hyperglycemia"** is defined as the condition in which a subject has a 2 hour postprandial blood glucose or serum glucose concentration greater than 200 mg/dL (11.11 mmol/L).

The term **"impaired fasting blood glucose"** or **"IFG"** is defined as the condition in which a subject has a fasting blood glucose concentration or fasting serum glucose concentration in a range from 100 to 125 mg/dl (i.e. from 5.6 to 6.9 mmol/l), in particular greater than 110 mg/dL and less than 126 mg/dl (7.00 mmol/L). A subject with "normal fasting glucose" has a fasting glucose concentration smaller than 100 mg/dl, i.e. smaller than 5.6 mmol/l.

The term **"impaired glucose tolerance"** or **"IGT"** is defined as the condition in which a subject has a 2 hour postprandial blood glucose or serum glucose concentration greater than 140 mg/dl (7.78 mmol/L) and less than 200 mg/dL (11.11 mmol/L). The abnormal glucose tolerance, i.e. the 2 hour postprandial blood glucose or serum glucose concentration can be measured as the blood sugar level in mg of glucose per dL of plasma 2 hours after taking 75 g of glucose after a fast. A subject with "normal glucose tolerance" has a 2 hour postprandial blood glucose or serum glucose concentration smaller than 140 mg/dl (7.78 mmol/L).

The term **"hyperinsulinemia"** is defined as the condition in which a subject with insulin resistance, with or without euglycemia, has fasting or postprandial serum or plasma insulin concentration elevated above that of normal, lean individuals without insulin resistance, having a waist-to-hip ratio < 1.0 (for men) or < 0.8 (for women).

The terms "insulin-sensitizing", "insulin resistance-improving" or "insulin resistance-lowering" are synonymous and used interchangeably.

The term **"insulin resistance"** is defined as a state in which circulating insulin levels in excess of the normal response to a glucose load are required to maintain the euglycemic state (Ford ES, et al. JAMA. (2002) 287:356-9). A method of determining insulin resistance is the euglycaemic-hyperinsulinaemic clamp test. The ratio of insulin to glucose is determined within the scope of a combined insulin-glucose infusion technique. There is found to be insulin resistance if the glucose absorption is below the 25th percentile of the background population investigated (WHO definition). Rather less laborious than the clamp test are so called minimal models in which, during an intravenous glucose tolerance test, the insulin and glucose concentrations in the blood are measured at fixed time intervals and from these the insulin resistance is calculated. With this method, it is not possible to distinguish between hepatic and peripheral insulin resistance.

Furthermore, insulin resistance, the response of a patient with insulin resistance to therapy, insulin sensitivity and hyperinsulinemia may be quantified by assessing the "homeostasis model assessment to insulin resistance (HOMA-IR)" score, a reliable indicator of insulin resistance (Katsuki A, et al. Diabetes Care 2001; 24: 362-5). Further reference is made to methods for the determination of the HOMA-index for insulin sensitivity *(*Matthews et al., Diabetologia 1985, 28: 412-19), of the ratio of intact proinsulin to insulin (Forst et al., Diabetes 2003, 52(Suppl.1): A459*)* and to an euglycemic clamp study. In addition, plasma adiponectin levels can be monitored as a potential surrogate of insulin sensitivity. The estimate of insulin resistance by the homeostasis assessment model (HOMA)-IR score is calculated with the formula (Galvin P, et al. Diabet Med 1992;9:921-8):

$$\text{HOMA-IR} = [\text{fasting serum insulin } (\mu U/mL)] \times [\text{fasting plasma glucose}(mmol/L)/22.5]$$

As a rule, other parameters are used in everyday clinical practice to assess insulin resistance. Preferably, the patient's triglyceride concentration is used, for example, as increased triglyceride levels correlate significantly with the presence of insulin resistance.

Patients with a predisposition for the development of IGT or IFG or type 2 diabetes are those having euglycemia with hyperinsulinemia and are by definition, insulin resistant. A typical patient with insulin resistance is usually overweight

or obese. If insulin resistance can be detected, this is a particularly strong indication of the presence of pre-diabetes. Thus, it may be that in order to maintain glucose homoeostasis a person needs 2-3 times as much insulin as a healthy person, without this resulting in any clinical symptoms.

The methods to investigate the **function of pancreatic beta-cells** are similar to the above methods with regard to insulin sensitivity, hyperinsulinemia or insulin resistance: An improvement of beta-cell function can be measured for example by determining a HOMA-index for beta-cell function *(*Matthews et al., Diabetologia 1985, 28: 412-19*),* the ratio of intact proinsulin to insulin *(*Forst et al., Diabetes 2003, 52(Suppl.1): A459*),* the insulin/C-peptide secretion after an oral glucose tolerance test or a meal tolerance test, or by employing a hyperglycemic clamp study and/or minimal modeling after a frequently sampled intravenous glucose tolerance test (Stumvoll et al., Eur J Clin Invest 2001, 31: 380-81).

The term **"pre-diabetes"** is the condition wherein an individual is pre-disposed to the development of type 2 diabetes. Pre-diabetes extends the definition of impaired glucose tolerance to include individuals with a fasting blood glucose within the high normal range ≥ 100 mg/dL (J. B. Meigs, et al. Diabetes 2003; 52:1475-1484) and fasting hyperinsulinemia (elevated plasma insulin concentration). The scientific and medical basis for identifying pre-diabetes as a serious health threat is laid out in a Position Statement entitled "The Prevention or Delay of Type 2 Diabetes" issued jointly by the American Diabetes Association and the National Institute of Diabetes and Digestive and Kidney Diseases (Diabetes Care 2002; 25:742-749).

Individuals likely to have insulin resistance are those who have two or more of the following attributes: 1) overweight or obese, 2) high blood pressure, 3) hyperlipidemia, 4) one or more $1^{st}$ degree relative with a diagnosis of IGT or IFG or type 2 diabetes. Insulin resistance can be confirmed in these individuals by calculating the HOMA-IR score. For the purpose of this invention, insulin resistance is defined as the clinical condition in which an individual has a HOMA-IR score > 4.0 or a HOMA-IR score above the upper limit of normal as defined for the laboratory performing the glucose and insulin assays.

The term **"type 2 diabetes"** is defined as the condition in which a subject has a fasting blood glucose or serum glucose concentration greater than 125 mg/dL (6.94 mmol/L). The measurement of blood glucose values is a standard procedure in routine medical analysis. If a glucose tolerance test is carried out, the blood sugar level of a diabetic will be in excess of 200 mg of glucose per dL (11.1 mmol/l) of plasma 2 hours after 75 g of glucose have been taken on an empty stomach. In a glucose tolerance test 75 g of glucose are administered orally to the patient being tested after 10-12 hours of fasting and the blood sugar level is recorded immediately before taking the glucose and 1 and 2 hours after taking it. In a healthy subject, the blood sugar level before taking the glucose will be between 60 and 110 mg per dL of plasma, less than 200 mg per dL 1 hour after taking the glucose and less than 140 mg per dL after 2 hours. If after 2 hours the value is between 140 and 200 mg, this is regarded as abnormal glucose tolerance.

The term **"late stage type 2 diabetes mellitus"** includes patients with a secondary drug failure, indication for insulin therapy and progression to micro- and macrovascular complications e.g. diabetic nephropathy, or coronary heart disease (CHD).

The term **"HbA1c"** refers to the product of a non-enzymatic glycation of the haemoglobin B chain. Its determination is well known to one skilled in the art. In monitoring the treatment of diabetes mellitus the HbA1c value is of exceptional importance. As its production depends essentially on the blood sugar level and the life of the erythrocytes, the HbA1c in the sense of a "blood sugar memory" reflects the average blood sugar levels of the preceding 4-6 weeks. Diabetic patients whose HbA1c value is consistently well adjusted by intensive diabetes treatment (i.e. < 6.5 % of the total haemoglobin in the sample), are significantly better protected against diabetic microangiopathy. For example, metformin on its own achieves an average improvement in the HbA1c value in the diabetic of the order of 1.0 - 1.5 %. This reduction of the HbA1C value is not sufficient in all diabetics to achieve the desired target range of < 6.5 % and preferably < 6 % HbA1c.

The term **"insufficient glycemic control"** or "inadequate glycemic control" in the scope of the present invention means a condition wherein patients show HbA1c values above 6.5 %, in particular above 7.0 %, even more preferably above 7.5 %, especially above 8 %.

The **"metabolic syndrome",** also called "syndrome X" (when used in the context of a metabolic disorder), also called the "dysmetabolic syndrome" is a syndrome complex with the cardinal feature being insulin resistance (Laaksonen DE, et al. Am J Epidemiol 2002;156:1070-7). According to the ATP III/NCEP guidelines (Executive Summary of the Third Report of the National Cholesterol Education Program (NCEP) Expert Panel on Detection, Evaluation, and Treatment of High Blood Cholesterol in Adults (Adult Treatment Panel III) JAMA: Journal of the American Medical Association (2001) 285:2486-2497), diagnosis of the metabolic syndrome is made when three or more of the following risk factors are present:

1. Abdominal obesity, defined as waist circumference > 40 inches or 102 cm in men, and > 35 inches or 94 cm in women; or with regard to a Japanese ethnicity or Japanese patients defined as waist circumference ≥ 85 cm in men and ≥ 90 cm in women;
2. Triglycerides: ≥ 150 mg/dL

3. HDL-cholesterol < 40 mg/dL in men
4. Blood pressure ≥ 130/85 mm Hg (SBP ≥ 130 or DBP ≥ 85)
5. Fasting blood glucose ≥ 100 mg/dL

The NCEP definitions have been validated (Laaksonen DE, et al. Am J Epidemiol. (2002) 156:1070-7). Triglycerides and HDL cholesterol in the blood can also be determined by standard methods in medical analysis and are described for example in Thomas L (Editor): "Labor und Diagnose", TH-Books Verlagsgesellschaft mbH, Frankfurt/Main, 2000.

According to a commonly used definition, **hypertension** is diagnosed if the systolic blood pressure (SBP) exceeds a value of 140 mm Hg and diastolic blood pressure (DBP) exceeds a value of 90 mm Hg. If a patient is suffering from manifest diabetes it is currently recommended that the systolic blood pressure be reduced to a level below 130 mm Hg and the diastolic blood pressure be lowered to below 80 mm Hg.

The definitions of **NODAT** (new onset diabetes after transplantation) and **PTMS** (post-transplant metabolic syndrome) follow closely that of the American Diabetes Association diagnostic criteria for type 2 diabetes, and that of the International Diabetes Federation (IDF) and the American Heart Association/National Heart, Lung, and Blood Institute, for the metabolic syndrome. NODAT and/or PTMS are associated with an increased risk of microand macrovascular disease and events, graft rejection, infection, and death. A number of predictors have been identified as potential risk factors related to NODAT and/or PTMS including a higher age at transplant, male gender, the pre-transplant body mass index, pre-transplant diabetes, and immunosuppression.

The term **"gestational diabetes"** (diabetes of pregnancy) denotes a form of the diabetes which develops during pregnancy and usually ceases again immediately after the birth. Gestational diabetes is diagnosed by a screening test which is carried out between the 24th and 28th weeks of pregnancy. It is usually a simple test in which the blood sugar level is measured one hour after the administration of 50 g of glucose solution. If this 1 h level is above 140 mg/dl, gestational diabetes is suspected. Final confirmation may be obtained by a standard glucose tolerance test, for example with 75 g of glucose.

The term **"hyperuricemia"** denotes a condition of high serum total urate levels. In human blood, uric acid concentrations between 3.6 mg/dL (ca. 214 μmol/L) and 8.3 mg/dL (ca. 494 μmol/L) are considered normal by the American Medical Association. High serum total urate levels, or hyperuricemia, are often associated with several maladies. For example, high serum total urate levels can lead to a type of arthritis in the joints kown as gout. Gout is a condition created by a build up of monosodium urate or uric acid crystals on the articular cartilage of joints, tendons and surrounding tissues due to elevated concentrations of total urate levels in the blood stream. The build up of urate or uric acid on these tissues provokes an inflammatory reaction of these tissues. Saturation levels of uric acid in urine may result in kidney stone formation when the uric acid or urate crystallizes in the kidney. Additionally, high serum total urate levels are often associated with the so-called metabolic syndrome, including cardiovascular disease and hypertension.

The term **"hyponatremia"** denotes a condition of a positive balance of water with or without a deficit of sodium, which is recognized when the plasma sodium falls below the level of 135 mml/L. Hyponatremia is a condition which can occur in isolation in individuals that overconsume water; however, more often hyponatremia is a complication of medication or other underlying medical condition that leas to a diminished excretion of water. Hyponatremia may lead to water intoxication, which occurs when the normal tonicity of extracellular fluid falls below the safe limit, due to retention of excess water. Water intoxication is a potentially fatal disturbance in brain function. Typical symptoms of water intoxication include nausea, vomiting, headache and malaise.

The term **"SGLT2 inhibitor"** in the scope of the present invention relates to compounds, in particular to glucopyranosyl-derivatives, i.e. compounds having a glucopyranosyl-moiety, which show an inhibitory effect on the sodium-glucose transporter 2 (SGLT2), in particular the human SGLT2. The inhibitory effect on hSGLT2 measured as IC50 is prerably below 1000 nM, even more preferably below 100 nM, most preferably below 50 nM. The inhibitory effect on hSGLT2 can be determined by methods known in the literature, in particular as described in the application WO 2005/092877 or WO 2007/093610 (pages 23/24). The term "SGLT2 inhibitor" also comprises any pharmaceutically acceptable salts thereof, hydrates and solvates thereof, including the respective crystalline forms.

The terms **"treatment"** and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy.

The terms **"prophylactically treating",** "preventivally treating" and "preventing" are used interchangeably and comprise a treatment of patients at risk to develop a condition mentioned hereinbefore, thus reducing said risk.

The term **"tablet"** comprises tablets without a coating and tablets with one or more coatings. Furthermore the "term" tablet comprises tablets having one, two, three or even more layers and press-coated tablets, wherein each of the beforementioned types of tablets may be without or with one or more coatings. The term "tablet" also comprises

mini, melt, chewable, effervescent and orally disintegrating tablets.

The terms **"pharmacopoe"** and **"pharmacopoeias"** refer to standard pharmacopoeias such as the "USP 31-NF 26 through Second Supplement" (United States Pharmacopeial Convention) or the "European Pharmacopoeia 6.3" (European Directorate for the Quality of Medicines and Health Care, 2000-2009).

**Detailed Description**

Any references in the description to methods of treatment refer to the SGLT2 inhibitor for use in a method of treatment in accordance with the claims.

In one aspect, the SGLT2 inhibitor is administered as defined hereinbefore and hereinafter. In one aspect, the subject is a patient having type 2 diabetes mellitus.

In one aspect the SGLT-2 inhibitor is empagliflozin (compound (1.9)).

The present invention provides the advantage of avoiding calorie restrictions protocols, which for example lead to calorie restriction or intermittent fasting, while still providing the health benefits derived from applying such protocols to the subject.

Calorie restriction or intermittent fasting, which also trigger elevation of ketone bodies in the blood, exhibit health benefit including increased lifespan expectancy in a subject applying such calorie restriction or intermittent fasting.

Other (non-claimed) benefits in the context of the present invention can include the prevention of seizures by enhancing brain energy production and the treament of epilepsy.

Metabolic myopathies

Patients with metabolic myopathies have underlying deficiencies of energy production in muscle due to a wide variety of defects. These include defects in lipid, glycogen, glucose, adenine nucleotide, and mitochondrial metabolism.

Metabolic myopathies and mitochondrial myopathies represent a group of heterogeneous genetic disorders that cause deficiencies of energy production in muscle. Muscles contraction depends on the chemical energy of ATP and several biochemical processes within the muscle cell to maintain and supply ATP to support muscle contraction. The three major pathways that supply ATP to meet the energy demands of exercising muscles are:

a) Glycogen metabolism: Glycogen is the main form of carbohydrate storage in the muscle. When energy is required for intensive and intermittent muscle contraction, glycogen is degraded to glucose (glycogenosis) to fuel the glycolysis and produce pyruvate which enters mitochondria to feed the Krebs cycle and produce ATP via mitochondria respiratory chain. Any disturbance in either the synthesis or the degradation of glycogen, in the glycolysis could results in glycogen storage disease.

b) Lipid metabolism: Long chain fatty acids are the major source of energy for skeletal muscle during sustained exercise or fasting. The passage of fatty acid through the mitochondria membrane, for beta oxidation to acetyl-CoA to fuel the Krebs cycle, requires their binding with carnitine for transport mediated by acyl-carnitine translocase and carnitine palmitoyl transferases (CPTs) I.

c) Mitochondrial function: Once in the mitochondria, substrates derived from glycogen and glucose pathway (pyruvate), and from fatty acid and beta oxidation are turned into acetyl coenzyme A, which feeds into Krebs cycle. In this critical cycle, production of intermediates molecules, NADH and $FADH_2$, will deliver the electrons to the mitochondrial respiratory chain to produce ATP and $H_2O$.

Defects in any of these pathways-glycogen catabolism (glycogenolysis and glycolysis), fatty acid oxidation, Krebs cycle, or mitochondrial respiratory chain and oxidative phosphorylation may cause human disorders that often predominantly affect muscle because of its high energy requirements, particularly during exercise.

SGLT-2 inhibitors, for example empaglifozin, decrease blood glucose independently of the insulin pathway via inhibition of the renal sodium-dependent glucose cotransporters 2 (SGLT2). By triggering the excretion of glucose in urine (glucosuria), a SGLT-2 inhibitor triggers a diminution in blood glucose associated with a diminution in insulin level. These effects trigger the mobilization of fat and the activation of the ketogenic pathway in the liver to produce and deliver ketones (especially acetoacetate and ß-hydroxybutyrate) in blood stream. These two ketone bodies represent another source of substrate to produce energy within different organs including skeletal muscle. These two energy substrates can enter freely in mitochondria to be oxidized to produce ATP. They thus constitute an alternative source of energy in patients with altered utilization of glycogen, glucose and fatty acid to produce energy necessary to muscle contraction. Among the energy substrates, ß-hydroxybutyrate offers also the advantage of being a more efficient energetic substrate in comparison to fat or glucose by delivering ATP molecule containing more free energy per unit of oxygen consumed necessary for muscle contraction. In addition, the augmentation in hematocrit induced by SGLT-2 inhibitors and the associated increase availability of oxygen at the level of mitochondria for energy production provide a powerful synergy

with the preferential use of ketones to fuel the muscle in energy for contraction, despite the blockage of other pathways in patients with metabolic myopathies.

Accordingly, in one aspect, the present invention provides a method of treating a metabolic myopathy in a patient, for example a metabolic myophathy from a glycogen or lipid metabolism disorder, comprising administering to the patient the SGLT-2 inhibitor. In one embodiment, the method replaces or mimicks a specific diet to increase energy production in organs, especially in muscles. In one embodiment, the method is in addition to a specific diet to increase energy production in organs, especially in muscles.

In one aspect, the present invention provides a method of delaying or slowing down the progression of a metabolic myopathy in a patient, for example a metabolic myophathy from a glycogen or lipid metabolism disorder, comprising administering to the patient the SGLT-2 inhibitor. In one embodiment, the method replaces or mimicks a specific diet to increase energy production in organs, especially in muscles. In one embodiment, the method is in addition to a specific diet to increase energy production in organs, especially in muscles.

In one aspect, a metabolic myopathy according to the present invention is a glycogen storage disease (GSD), a fatty acid oxidation defect (FAODs) or a mitochondrial myopathy.

Glycogen Storages Diseases (GSDs):

Glycogen is the main source of energy during brief exercise while free fatty acids are the most important source of fuel during prolonged exercise. Hence, muscle cramps during strenuous brief exercise are the hallmark of glycogen storage diseases (eg McArdle disease). These conditions results from a variety of enzymatic defects that perturb glycogen synthesis, (glycogenosis) or its degradation to glucose (glycolysis). A GSD is for example one of the following eleven disorders (designated from I to XI) of glycogen metabolism:

- GSD I - Glucose-6-phosphatase deficiency; Von Gierke Disease
    - GSD IB/IC; Transporteur du glucose-6-phosphatase
- GSD II - Acid maltase deficiency (AMD); Pompe disease.
    - GSD IIB; Lysosomal-Associated Membrane Protein 2; Danon disease
- GSD III - Debrancher enzyme deficiency; Cori-Forbes disease.
- GSD IV - Brancher enzyme deficiency; Andersen disease.
- GSD V - Muscle phosphorylase deficiency; McArdle disease.
- GSD VI - Liver phosphorylase deficiency; Hers disease
- GSD VII - Phosphofructokinase deficiency; Tarui disease.
- GSD VIII - Phosphorylase b kinase deficiency.
- GSD type IX - Phosphoglycerate kinase deficiency.
- GSD X - Phosphoglycerate mutase deficiency.
- GSDXI - Lactate dehydrogenase deficiency.
- GSD XII - Aldolase A deficiency.

Accordingly, in one aspect, the present invention provides a method of treating, or delaying or slowing down the progression of, any one of the above Glycogen Storages Diseases (GSDs) comprising administering to a patient the SGLT-2 inhibitor, for example empagliflozin.

Lipid metabolism disorders:

Myopathies resulting from a disorder of lipid metabolism include:

- Carnitine deficiency syndromes
- Fatty acid transport defects
- Defects of beta-oxidation enzyme

The most common lipid metabolism disorders include Carnitine palmitoyltransferase II deficiency (CPTII), trifunctional protein deficiency (TFP) and very long-chain acyl-CoA deshydrogenase deficiency (VLCAD).

Around 60 different diseases causing mutations within the CPTII gene has been reported triggering rhadbdomyolysis and myoglobinuria.

Accordingly, in one aspect, the present invention provides a method of treating, or delaying or slowing down the progression of, any one of the above lipid metabolism disorders comprising administering to a patient the SGLT-2 inhibitor, for example empagliflozin.

Mitochondrial myopathies:

These myopathies comprise a diverse group of multisystem disease caused by hereditary abnormalities of the mitochondrial respiratory chain and produce a plethora of clinical phenotypes. One of the most common symptoms affecting patients with mitochondrial diseases is exercise intolerance due to premature fatigue with activities as mild as walking up a single flight of stairs. After a short rest, patients usually can resume their activity, but symptoms recur. Patients with mitochondrial disease often report subjective heaviness or burning of muscles with exertion but, in contrast to patients with glycogenoses, they typically do not manifest stiffness, cramps, or second wind phenomenon.

Some of the more common mitochondrial muscle disorders includes MELAS (Mitochondrial Encephalomyopathy, Lactic Acidosis, and Stroke-like episodes), MERRF (Myoclonic Epilepsy with Ragged Red Fibers), mtDNA deletion, Kearns-Sayre syndrome, complex I deficiency, cytochrome b mutations, cytochrome c oxidase mutations.

Accordingly, in one aspect, the present invention provides a method of treating, or delaying or slowing down the progression of, any one of the above mitochondrial myopathies comprising administering to a patient the SGLT-2 inhibitor, for example empagliflozin.

In one aspect, the administration of the SGLT-2 inhibitor to one of the above patients leads to better tolerance to exercise and/or less fatigability and cramps. In one aspect, the administration of the SGLT-2 inhibitor to one of these patients reduces muscle damages and/or associated rhadbdomyolysis and myoglobinuria. These effects should improve the quality of life of these patients.

In a (non-claimed) aspect, a disease according to the present disclosure is Glucose Transporter Type-1 Deficiency Syndrome (Glut1 DS). This disease is characterized by the inability to transport glucose into the brain. Accordingly, in one aspect, the present disclosure provides a method of treating, or delaying or slowing down the progression of Glucose Transporter Type-1 Deficiency Syndrome (Glut1 DS) comprising administering to a patient a SGLT-2 inhibitor, for example empagliflozin.

In one aspect, the administration of the SGLT2 inhibitor can improve the quality of life of a patient with a metabolic myopathy, for example by improving the energy and oxygen supply to organs, in particular to muscles to improve contraction and resistance to exercise.

The aspects according to the present invention, in particular the pharmaceutical compositions, methods and uses, refer to SGLT2 inhibitors as defined hereinbefore and hereinafter.

The SGLT2 inhibitor is selected from a glucopyranosyl-substituted benzene derivative of the formula (I)

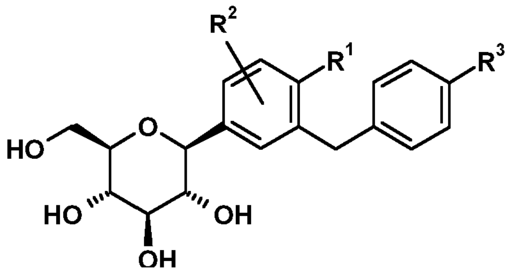

I

wherein $R^1$ denotes Cl or methyl; $R^2$ denotes H, methyl, methoxy or hydroxy and $R^3$ denotes ethyl, cyclopropyl, ethynyl, ethoxy, (*R*)-tetrahydrofuran-3-yloxy or (*S*)-tetrahydrofuran-3-yloxy; or a prodrug of one of the beforementioned SGLT2 inhibitors.

Compounds of the formula (I) and methods of their synthesis are described for example in the following patent applications: WO 2005/092877.

In the above glucopyranosyl-substituted benzene derivatives of the formula (I) the following definitions of the substituents are preferred.

Preferably $R^1$ denotes chloro.

Preferably $R^2$ denotes H.

Preferably $R^3$ denotes *(S)*-tetrahydrofuran-3-yloxy.

A preferred glucopyranosyl-substituted benzene derivatives of the formula (I) is compound (1.9), also referred to as empagliflozin:

| | | |
|---|---|---|
| (I.9) | 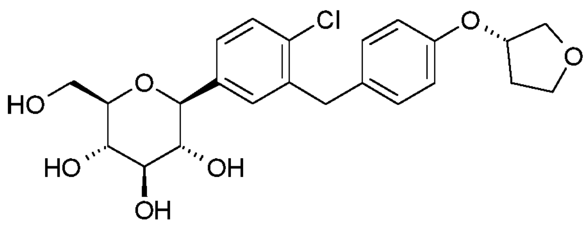 1-chloro-4-(β-D-glucopyranos-1-yl)-2-[4-((*S*)-tetrahydrofuran-3-yloxy)-benzyl]-benzene, |

According to this invention, it is to be understood that the definitions of the above listed glucopyranosyl-substituted benzene derivatives of the formula (I) also comprise their hydrates, solvates and polymorphic forms thereof, and prodrugs thereof. With regard to the preferred compound (1.9) an advantageous crystalline form is described in the international patent applciation WO 2006/117359. These crystalline forms possess good solubility properties which enable a good bioavailability of the SGLT2 inhibitor. Furthermore, the crystalline forms are physico-chemically stable and thus provide a good shelf-life stability of the pharmaceutical composition.

In the following the suitable excipients and carriers in the pharmaceutical compositions according to the invention are described in further detail.

In the following, preferred ranges of the amount of the glucopyranosyl-substituted benzene derivative to be employed in the pharmaceutical dosage form according to this invention are described. These ranges refer to the amounts to be administered per day with respect to an adult patient, in particular to a human being, for example of approximately 70 kg body weight, and can be adapted accordingly with regard to an administration 2, 3, 4 or more times daily and with regard to other routes of administration and with regard to the age of the patient. The ranges of the dosage and amounts are calculated for the active ingredient.

A preferred amount of the glucopyranosyl-substituted benzene derivative, in particular the compound (1.9) or its crystalline form (I.9X) is in a range from 0.5 to 100 mg, preferably from 0.5 to 50 mg, even more preferably from 1 to 25 mg, even more preferably 5 to 25 mg. Preferred dosages of the glucopyranosyl-substituted benzene derivative are for example 1 mg, 2 mg, 2.5 mg, 5 mg, 7.5 mg, 10 mg, 12.5 mg, 15 mg, 20 mg, 25 mg and 50 mg, in particular 10 mg and 25 mg.

A pharmaceutical composition according to the present invention may be comprised in a tablet, a capsule or a film-coated tablet,

In one embodiment, a tablet comprising a pharmaceutical composition according to the present invention comprises a lubricant, such as magnesium stearate. Such lubricant may be present in a concentration of 0.25 - 2 % in said tablet.

In one embodiment, a tablet comprising a pharmaceutical composition according to the present invention comprises a glidant, such as colloidal silicon dioxide. Such glidant may be present in a concentration of 0.25 - 2 % in said tablet.

A tablet according to the invention may be film-coated. Typically a film coat represents 2-5% by weight of the total composition and comprises preferably a film-forming agent, a plasticizer, a glidant and optionally one or more pigments. An exemplary coat composition may comprise hydroxypropylmethyl-cellulose (HPMC), polyethylene glycol (PEG), talc, titanium dioxide and optionally iron oxide, including iron oxide red and/or yellow.

A dosage form according to this invention, such as a tablet, capsule or film-coated tablet, may be prepared by methods well-known to the one skilled in the art.

Suitable methods of manufacturing a tablet include compression of the pharmaceutical composition in the form of a powder, i.e. direct compression, or compression of the pharmaceutical composition in the form of granules, and if needed with additional excipients.

Granules of the pharmaceutical composition according to the invention may be prepared by methods well-known to the one skilled in the art. Preferred methods for the granulation of the active ingredients together with the excipients include wet granulation, for example high shear wet granulation and fluidized bed wet granulation, dry granulation, also called roller compaction.

When this invention refers to patients requiring treatment or prevention, it relates primarily to treatment and prevention in humans, but the pharmaceutical composition may also be used accordingly in veterinary medicine in mammals. In the scope of this invention adult patients are preferably humans of the age of 18 years or older. Also in the scope of this invention, patients are adolescent humans, i.e. humans of age 10 to 17 years, preferably of age 13 to 17 years. Also in the scope of this invention, patients are human childran, i.e. humans of age of less than 10 years, preferably of age 6 to 9 years. It is assumed that in a adolescent population the administration of the pharmaceutical

composition according to the invention a very good HbA1c lowering and a very good lowering of the fasting plasma glucose can be seen. In addition it is assumed that in an adolescent population, in particular in overweight and/or obese patients, a pronounced weight loss can be observed.

As described hereinbefore by the administration of the pharmaceutical composition according to this invention and in particular in view of the high SGLT2 inhibitory activity of the SGLT2 inhibitors therein, excessive blood glucose is excreted through the urine of the patient, so that no gain in weight or even a reduction in body weight may result. Therefore, a treatment or prophylaxis according to this invention is advantageously suitable in those patients in need of such treatment or prophylaxis who are diagnosed of one or more of the conditions selected from the group consisting of overweight and obesity, in particular class I obesity, class II obesity, class III obesity, visceral obesity and abdominal obesity. In addition a treatment or prophylaxis according to this invention is advantageously suitable in those patients in which a weight increase is contraindicated. The pharmaceutical composition as well as the methods according to the present invention allow a reduction of the HbA1c value to a desired target range, for example < 7 % and preferably < 6.5 %, for a higher number of patients and for a longer time of therapeutic treatment compared with a corresponding monotherapy or a therapy using only two of the combination partners.

The pharmaceutical composition according to this invention and in particular the SGLT2 inhibitor therein exhibits a very good efficacy with regard to glycemic control, in particular in view of a reduction of fasting plasma glucose, postprandial plasma glucose and/or glycosylated hemoglobin (HbA1c). By administering a pharmaceutical composition according to this invention, a reduction of HbA1c equal to or greater than preferably 0.5 %, even more preferably equal to or greater than 1.0 % can be achieved and the reduction is particularly in the range from 1.0 % to 2.0 %.

Furthermore, the method and/or use according to this invention is advantageously applicable in those patients who show one, two or more of the following conditions:

(a) a fasting blood glucose or serum glucose concentration greater than 100 mg/dL, in particular greater than 125 mg/dL;
(b) a postprandial plasma glucose equal to or greater than 140 mg/dL;
(c) an HbA1c value equal to or greater than 6.5 %, in particular equal to or greater than 7.0 %, especially equal to or greater than 7.5 %, even more particularly equal to or greater than 8.0 %.

The present disclosure also discloses the (non-claimed) use of the pharmaceutical composition for improving glycemic control in patients having type 2 diabetes or showing first signs of pre-diabetes. Thus, the disclosure also includes diabetes prevention. If therefore a pharmaceutical composition according to this disclosure is used to improve the glycemic control as soon as one of the above-mentioned signs of pre-diabetes is present, the onset of manifest type 2 diabetes mellitus can be delayed or prevented.

Furthermore, the pharmaceutical composition according to this disclosure is particularly suitable in the treatment of patients with insulin dependency, i.e. in patients who are treated or otherwise would be treated or need treatment with an insulin or a derivative of insulin or a substitute of insulin or a formulation comprising an insulin or a derivative or substitute thereof. These patients include patients with diabetes type 2 and patients with diabetes type 1.

Therefore, according to a (non-claimed) preferred embodiment of the present disclosure, there is provided a method for improving glycemic control and/or for reducing of fasting plasma glucose, of postprandial plasma glucose and/or of glycosylated hemoglobin HbA1c in a patient in need thereof who is diagnosed with impaired glucose tolerance (IGT), impaired fasting blood glucose (IFG) with insulin resistance, with metabolic syndrome and/or with type 2 or type 1 diabetes mellitus characterized in that an SGLT2 inhibitor as defined hereinbefore and hereinafter is administered to the patient.

According to another (non-claimed) preferred embodiment of the present disclosure, there is provided a method for improving gycemic control in patients, in particular in adult patients, with type 2 diabetes mellitus as an adjunct to diet and exercise.

It can be found that by using a pharmaceutical composition according to this invention, an improvement of the glycemic control can be achieved even in those patients who have insufficient glycemic control in particular despite treatment with an antidiabetic drug, for example despite maximal recommended or tolerated dose of oral monotherapy with metformin. A maximal recommended dose with regard to metformin is for example 2000 mg per day or 850 mg three times a day or any equivalent thereof.

Therefore, the method and/or use according to this invention is advantageously applicable in those patients who show one, two or more of the following conditions:

(a) insufficient glycemic control with diet and exercise alone;
(b) insufficient glycemic control despite oral monotherapy with metformin, in particular despite oral monotherapy at a maximal tolerated dose of metformin;
(c) insufficient glycemic control despite oral monotherapy with another antidiabetic agent, in particular despite oral

monotherapy at a maximal tolerated dose of the other antidiabetic agent.

The lowering of the blood glucose level by the administration of an SGLT2 inhibitor according to this invention is insulin-independent. Therefore, a pharmaceutical composition according to this invention is particularly suitable in the (non-claimed) treatment of patients who are diagnosed having one or more of the following conditions

- insulin resistance,
- hyperinsulinemia,
- pre-diabetes,
- type 2 diabetes mellitus, particular having a late stage type 2 diabetes mellitus,
- type 1 diabetes mellitus.

Furthermore, a pharmaceutical composition according to this invention is particularly suitable in the (non-claimed) treatment of patients who are diagnosed having one or more of the following conditions

(a) obesity (including class I, II and/or III obesity), visceral obesity and/or abdominal obesity,
(b) triglyceride blood level ≥ 150 mg/dL,
(c) HDL-cholesterol blood level < 40 mg/dL in female patients and < 50 mg/dL in male patients,
(d) a systolic blood pressure ≥ 130 mm Hg and a diastolic blood pressure ≥ 85 mm Hg,
(e) a fasting blood glucose level ≥ 100 mg/dL.

It is assumed that patients diagnosed with impaired glucose tolerance (IGT), impaired fasting blood glucose (IFG), with insulin resistance and/or with metabolic syndrome suffer from an increased risk of developing a cardiovascular disease, such as for example myocardial infarction, coronary heart disease, heart insufficiency, thromboembolic events. A glycemic control according to this disclosure may result in a reduction of the cardiovascular risks.

Furthermore, a pharmaceutical composition according to this invention is particularly suitable in the (non-claimed) treatment of patients after organ transplantation, in particular those patients who are diagnosed having one or more of the following conditions

(a) a higher age, in particular above 50 years,
(b) male gender;
(c) overweight, obesity (including class I, II and/or III obesity), visceral obesity and/or abdominal obesity,
(d) pre-transplant diabetes,
(e) immunosuppression therapy.

Furthermore, a pharmaceutical composition according to this invention is particularly suitable in the (non-claimed) treatment of patients who are diagnosed having one or more of the following conditions:

(a) hyponatremia, in particular chronical hyponatremia;
(b) water intoxication;
(c) water retention;
(d) plasma sodium concentration below 135 mmol/L.

The patient may be a diabetic or non-diabetic mammal, in particular human.

Furthermore, a pharmaceutical composition according to this invention is particularly suitable

in the (non-claimed) treatment of patients who are diagnosed having one or more of the following conditions:

(a) high serum uric acid levels, in particular greater than 6.0 mg/dL (357 μmol/L);
(b) a history of gouty arthritis, in particular recurrent gouty arthritis;
(c) kidney stones, in particular recurrent kidney stones;
(d) a high propensity for kidney stone formation.

A pharmaceutical composition according to this invention exhibits a good safety profile. Therefore, a treatment or prophylaxis according to this invention is advantageously possible in those patients for which the mono-therapy with another antidiabetic drug, such as for example metformin, is contraindicated and/or who have an intolerance against such drugs at therapeutic doses. In particular, a treatment or prophylaxis according to this invention may be advantageously possible in those patients showing or having an increased risk for one or more of the following disorders: renal insufficiency or diseases, cardiac diseases, cardiac failure, hepatic diseases, pulmonal diseases, catabolytic states

and/or danger of lactate acidosis, or female patients being pregnant or during lactation.

Furthermore, it can be found that the administration of a pharmaceutical composition according to this invention results in no risk or in a low risk of hypoglycemia. Therefore, a treatment or prophylaxis according to this invention is also advantageously possible in those patients showing or having an increased risk for hypoglycemia.

A pharmaceutical composition according to this invention is particularly suitable in the long term treatment or prophylaxis of the diseases and/or conditions as claimed.

The term "long term" as used hereinbefore and hereinafter indicates a treatment of or administration in a patient within a period of time longer than 12 weeks, preferably longer than 25 weeks, even more preferably longer than 1 year.

Therefore, a particularly preferred (non-claimed) aspect of the present disclosure provides a method for therapy, preferably oral therapy, for improvement, especially long term improvement, of glycemic control in patients with type 2 diabetes mellitus, especially in patients with late stage type 2 diabetes mellitus, in particular in patients additionally diagnosed of overweight, obesity (including class I, class II and/or class III obesity), visceral obesity and/or abdominal obesity.

It will be appreciated that the amount of the pharmaceutical composition according to this invention to be administered to the patient and required for use in treatment or prophylaxis according to the present invention will vary with the route of administration, the nature and severity of the condition for which treatment or prophylaxis is required, the age, weight and condition of the patient, concomitant medication and will be ultimately at the discretion of the attendant physician. In general, however, the SGLT2 inhibitor according to this invention is included in the pharmaceutical composition or dosage form in an amount sufficient that by its administration the glycemic control in the patient to be treated is improved.

For the (non-claimed) treatment of hyperuricemia or hyperuricemia associated conditions the SGLT2 inhibitor according to this invention is included in the pharmaceutical composition or dosage form in an amount sufficient that is sufficient to treat hyperuricemia without disturbing the patient's plasma glucose homeostasis, in particular without inducing hypoglycemia.

For the (non-claimed) treatment or prevention of kidney stones the SGLT2 inhibitor according to this invention is included in the pharmaceutical composition or dosage form in an amount sufficient that is sufficient to treat or prevent kidney stones without disturbing the patient's plasma glucose homeostasis, in particular without inducing hypoglycemia.

For the (non-claimed) treatment of hyponatremia and associated conditions the SGLT2 inhibitor according to this invention is included in the pharmaceutical composition or dosage form in an amount sufficient that is sufficient to treat hyponatremia or the associated conditions without disturbing the patient's plasma glucose homeostasis, in particular without inducing hypoglycemia.

In the following preferred ranges of the amount of the SGLT2 inhibitor to be employed in the pharmaceutical composition and the methods and uses according to this invention are described. These ranges refer to the amounts to be administered per day with respect to an adult patient, in particular to a human being, for example of approximately 70 kg body weight, and can be adapted accordingly with regard to an administration 2, 3, 4 or more times daily and with regard to other routes of administration and with regard to the age of the patient. Within the scope of the present invention, the pharmaceutical composition is preferably administered orally. Other forms of administration are possible and described hereinafter. Preferably the one or more dosage forms comprising the SGLT2 inhibitor is oral or usually well known.

In general, the amount of the SGLT2 inhibitor in the pharmaceutical composition and methods according to this invention is preferably the amount usually recommended for a monotherapy using said SGLT2 inhibitor.

The preferred dosage range of the SGLT2 inhibitor is in the range from 0.5 mg to 200 mg, even more preferably from 1 to 100 mg, most preferably from 1 to 50 mg per day. The oral administration is preferred. Therefore, a pharmaceutical composition may comprise the hereinbefore mentioned amounts, in particular from 1 to 50 mg or 1 to 25 mg. Particular dosage strengths (e.g. per tablet or capsule) are for example 1, 2.5, 5, 7.5, 10, 12.5, 15, 20, 25 or 50 mg of the SGLT2 inhibitor, such as a compound of the formula (I), in particular of the compound (1.9) or its crystalline form (I.9X). The application of the active ingredient may occur up to three times a day, preferably one or two times a day, most preferably once a day. Particular dosage strengths of empagliflozin (compound (1.9) are 10 mg or 25 mg once a day or 5 mg or 12.5 mg twice a day.

A pharmaceutical composition which is present as a separate or multiple dosage form, preferably as a kit of parts, is useful in combination therapy to flexibly suit the individual therapeutic needs of the patient.

According to a first embodiment a preferred kit of parts comprises a containment containing a dosage form comprising the SGLT2 inhibitor and at least one pharmaceutically acceptable carrier.

A further (non-claimed) aspect of the present disclosure is a manufacture comprising the pharmaceutical composition being present as separate dosage forms according to the present invention and a label or package insert comprising instructions that the separate dosage forms are to be administered in combination or alternation.

According to a first aspect a manufacture comprises (a) a pharmaceutical composition comprising a SGLT2 inhibitor according to the present invention and (b) a label or package insert which comprises instructions that the medicament is to be administered.

The desired dose of the pharmaceutical composition according to this invention may conveniently be presented in a once daily or as divided dose administered at appropriate intervals, for example as two, three or more doses per day.

The pharmaceutical composition may be formulated for oral, rectal, nasal, topical (including buccal and sublingual), transdermal, vaginal or parenteral (including intramuscular, subcutaneous and intravenous) administration in liquid or solid form or in a form suitable for administration by inhalation or insufflation. Oral administration is preferred. The formulations may, where appropriate, be conveniently presented in discrete dosage units and may be prepared by any of the methods well known in the art of pharmacy. All methods include the step of bringing into association the active ingredient with one or more pharmaceutically acceptable carriers, like liquid carriers or finely divided solid carriers or both, and then, if necessary, shaping the product into the desired formulation.

The pharmaceutical composition may be formulated in the form of tablets, granules, fine granules, powders, capsules, caplets, soft capsules, pills, oral solutions, syrups, dry syrups, chewable tablets, troches, effervescent tablets, drops, suspension, fast dissolving tablets, oral fast-dispersing tablets, etc..

The pharmaceutical composition and the dosage forms preferably comprises one or more pharmaceutical acceptable carriers which must be "acceptable" in the sense of being compatible with the other ingredients of the formulation and not deleterious to the recipient thereof. Examples of pharmaceutically acceptable carriers are known to the one skilled in the art.

Pharmaceutical compositions suitable for oral administration may conveniently be presented as discrete units such as capsules, including soft gelatin capsules, cachets or tablets each containing a predetermined amount of the active ingredient; as a powder or granules; as a solution, a suspension or as an emulsion, for example as syrups, elixirs or self-emulsifying delivery systems (SEDDS). The active ingredients may also be presented as a bolus, electuary or paste. Tablets and capsules for oral administration may contain conventional excipients such as binding agents, fillers, lubricants, disintegrants, or wetting agents. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, emulsifying agents, non-aqueous vehicles (which may include edible oils), or preservatives.

The pharmaceutical composition according to the disclosure may also be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulatory agents such as suspending, stabilizing and/or dispersing agents. Alternatively, the active ingredients may be in powder form, obtained by aseptic isolation of sterile solid or by lyophilisation from solution, for constitution with a suitable vehicle, e.g. sterile, pyrogen-free water, before use.

Pharmaceutical compositions suitable for rectal administration wherein the carrier is a solid are most preferably presented as unit dose suppositories. Suitable carriers include cocoa butter and other materials commonly used in the art, and the suppositories may be conveniently formed by admixture of the active compound(s) with the softened or melted carrier(s) followed by chilling and shaping in moulds.

The pharmaceutical compositions and methods according to this invention show advantageous effects in the treatment and prevention of those diseases and conditions as described hereinbefore. Advantageous effects may be seen for example with respect to efficacy, dosage strength, dosage frequency, pharmacodynamic properties, pharmacokinetic properties, fewer adverse effects, convenience, compliance, etc..

Methods for the manufacture of SGLT2 inhibitors according to this invention and of prodrugs thereof are known to the one skilled in the art. Advantageously, the compounds according to this invention can be prepared using synthetic methods as described in the literature, including patent applications as cited hereinbefore. Preferred methods of manufacture are described in the WO 2006/120208 and WO 2007/031548. With regard to compound (1.9) an advantageous crystalline form is described in the international patent application WO 2006/117359.

The active ingredients may be present in the form of a pharmaceutically acceptable salt. Pharmaceutically acceptable salts include, without being restricted thereto, such as salts of inorganic acid like hydrochloric acid, sulfuric acid and phosphoric acid; salts of organic carboxylic acid like oxalic acid, acetic acid, citric acid, malic acid, benzoic acid, maleic acid, fumaric acid, tartaric acid, succinic acid and glutamic acid and salts of organic sulfonic acid like methanesulfonic acid and p-toluenesulfonic acid. The salts can be formed by combining the compound and an acid in the appropriate amount and ratio in a solvent and decomposer. They can be also obtained by the cation or anion exchange from the form of other salts. The active ingredients or a pharmaceutically acceptable salt thereof may be present in the form of a solvate such as a hydrate or alcohol adduct.

Any of the above mentioned pharmaceutical compositions and methods within the scope of the invention may be tested by animal models known in the art. In the following, *in vivo* experiments are described which are suitable to evaluate pharmacologically relevant properties of pharmaceutical compositions and methods according to this invention. Pharmaceutical compositions and methods according to this invention can be tested in genetically hyperinsulinemic or

diabetic animals like db/db mice, ob/ob mice, Zucker Fatty (fa/fa) rats or Zucker Diabetic Fatty (ZDF) rats. In addition, they can be tested in animals with experimentally induced diabetes like HanWistar or Sprague Dawley rats pretreated with streptozotocin.

The (non-claimed) effect on glycemic control according to this invention can be tested after single dosing of the SGLT2 inhibitor in an oral glucose tolerance test in the animal models described hereinbefore. The time course of blood glucose is followed after an oral glucose challenge in overnight fasted animals. The pharmaceutical compositions according to the present invention significantly improve glucose excursion, for example compared to another monotherapy, as measured by reduction of peak glucose concentrations or reduction of glucose AUC. In addition, after multiple dosing of the SGLT2 inhibitor in the animal models described hereinbefore, the effect on glycemic control can be determined by measuring the HbA1c value in blood. The pharmaceutical compositions according to this invention significantly reduce HbA1c, for example compared to another monotherapy or compared to a dual-combination therapy.

The (non-claimed) improved independence from insulin of the treatment according to this invention can be shown after single dosing in oral glucose tolerance tests in the animal models described hereinbefore. The time course of plasma insulin is followed after a glucose challenge in overnight fasted animals.

The (non-claimed) increase in active GLP-1 levels by treatment according to this invention after single or multiple dosing can be determined by measuring those levels in the plasma of animal models described hereinbefore in either the fasting or postprandial state. Likewise, a reduction in glucagon levels in plasma can be measured under the same conditions.

The (non-claimed) effect of a SGLT2 inhibitor according to the present invention on beta-cell regeneration and neogenesis can be determined after multiple dosing in the animal models described hereinbefore by measuring the increase in pancreatic insulin content, or by measuring increased beta-cell mass by morphometric analysis after immunhistochemical staining of pancreatic sections, or by measuring increased glucose-stimulated insulin secretion in isolated pancreatic islets.

## Examples

### Example of Pharmaceutical Composition and Dosage Form

The following example of solid pharmaceutical compositions and dosage forms for oral administration serves to illustrate the present invention more fully without restricting it to the contents of the example. Further examples of compositions and dosage forms for oral administration, are described in WO 2010/092126. The term "active substance" denotes empagliflozin according to this invention, especially its crystalline form as described in WO 2006/117359 and WO 2011/039107.

Tablets containing 2.5mg, 5mg, 10mg, 25mg, 50mg of active substance

| **Active substance** | 2.5 mg/ per tablet | 5 mg/ per tablet | 10 mg/ per tablet | 25 mg/ per tablet | 50 mg/ per tablet |
|---|---|---|---|---|---|
| **Wet granulation** | | | | | |
| active substance | 2.5000 | 5.000 | 10.00 | 25.00 | 50.00 |
| Lactose Monohydrate | 40.6250 | 81.250 | 162.50 | 113.00 | 226.00 |
| Microcrystalline Cellulose | 12.5000 | 25.000 | 50.00 | 40.00 | 80.00 |
| Hydroxypropyl Cellulose | 1.8750 | 3.750 | 7.50 | 6.00 | 12.00 |
| Croscarmellose Sodium | 1.2500 | 2.500 | 5.00 | 4.00 | 8.00 |
| Purified Water | q.s. | q.s. | q.s. | q.s. | q.s. |
| Dry Adds | | | | | |
| Microcrystalline Cellulose | 3.1250 | 6.250 | 12.50 | 10.00 | 20.00 |
| Colloidal silicon dioxide | 0.3125 | 0.625 | 1.25 | 1.00 | 2.00 |
| Magnesium stearate | 0.3125 | 0.625 | 1.25 | 1.00 | 2.00 |
| Total core | 62.5000 | 125.000 | 250.00 | 200.00 | 400.00 |

(continued)

| Film Coating | | | | | |
|---|---|---|---|---|---|
| Film coating system | 2.5000 | 4.000 | 7.00 | 6.00 | 9.00 |
| Purified Water | q.s. | q.s. | q.s. | q.s. | q.s. |
| **Total** | **65.000** | **129.000** | **257.00** | **206.00** | **409.00** |

Details regarding the manufacture of the tablets, the active pharmaceutical ingredient, the excipients and the film coating system are described in WO 2010/092126, in particular in the Examples 5 and 6.

**Pharmacological examples**

A SGLT-2 inhibitor, for example empagliflozin, is assessed in animal models. The SGLT-2 inhibitor is administered to the animals and muscle performance and exercise tolerance are measured. Blood lactate at rest and during exercise is also measured.

The experiments can be carried out in animal models of metabolic myopathy.

## Claims

1. A SGLT-2 inhibitor for use in a method of treating, delaying or slowing down the progression of a metabolic myopathy in a patient, said method comprising administering to the patient the SGLT-2 inhibitor wherein the SGLT2 inhibitor is selected from a glucopyranosyl-substituted benzene derivative of the formula (I)

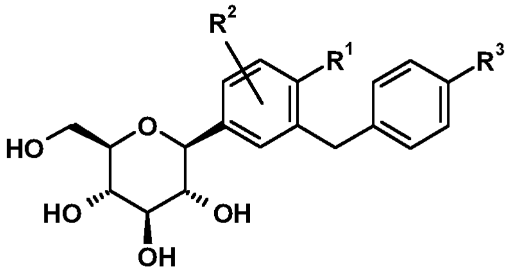

I

wherein $R^1$ denotes Cl or methyl; $R^2$ denotes H, methyl, methoxy or hydroxy and $R^3$ denotes ethyl, cyclopropyl, ethynyl, ethoxy, (*R*)-tetrahydrofuran-3-yloxy or (*S*)-tetrahydrofuran-3-yloxy.

2. The SGLT-2 inhibitor for use in a method of claim 1, wherein said metabolic myopathy is a metabolic myopathy from a glycogen or lipid metabolism disorder.

3. The SGLT-2 inhibitor for use in a method of claim 1, wherein said metabolic myopathy is a glycogen storage disease (GSD), a fatty acid oxidation defect (FAODs) or a mitochondrial myopathy.

4. The SGLT-2 inhibitor for use in a method of claim 1, 2 or 3 wherein the administration leads to better tolerance to exercise and/or less fatigability and cramps.

5. The SGLT-2 inhibitor for use in a method of claim 1, 2 or 3 wherein the administration reduces muscle damages and/or associated rhadbdomyolysis and myoglobinuria.

6. The SGLT-2 inhibitor for use in a method of any one of claims 1 to 5, wherein said patient is a patient having type 2 diabetes mellitus.

7. The SGLT-2 inhibitor for use in a method of any one of claims 1 to 6, wherein the SGLT-2 inhibitor is empagliflozin.

8. The SGLT-2 inhibitor for use in a method of any one of claims 1 to 7, wherein the dosage range of the SGLT-2 inhibitor is from 1 to 50 mg per day.

9. The SGLT-2 inhibitor for use in a method of claim 7, wherein the dosage strength of the SGLT-2 inhibitor empagliflozin is 10 mg or 25 mg once a day or 5 mg or 12.5 mg twice a day.

10. The SGLT-2 inhibitor for use in a method according to any one of claims 1 to 9, wherein said administration of the SGLT-2 inhibitor to the patient is in addition to a specific diet taken by the patient to increase energy production in organs, especially in muscles.

**Patentansprüche**

1. SGLT-2-Inhibitor zur Verwendung in einem Verfahren zum Behandeln, Verzögern oder Verlangsamen des Fortschreitens einer Stoffwechselmyopathie bei einem Patienten, wobei das Verfahren die Verabreichung des SGLT-2-Inhibitors an den Patienten umfasst, wobei der SGLT2-Inhibitor ausgewählt ist aus einem Glucopyranosylsubstituierten Benzolderivat der Formel (I)

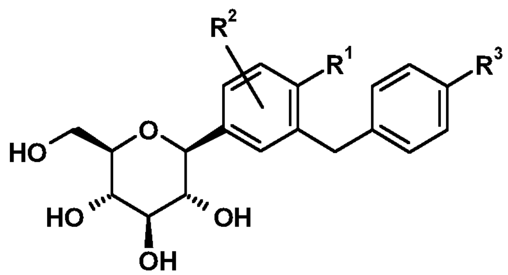

I

wobei $R^1$ Cl oder Methyl bezeichnet; $R^2$ bezeichnet H, Methyl, Methoxy oder Hydroxy und $R^3$ bezeichnet Ethyl, Cyclopropyl, Ethinyl, Ethoxy, *(R)*-Tetrahydrofuran-3-yloxy oder *(S)*-Tetrahydrofuran-3-yloxy.

2. SGLT-2-Inhibitor zur Verwendung in einem Verfahren nach Anspruch 1, wobei die Stoffwechselmyopathie eine Stoffwechselmyopathie aus einer Glycogen- oder Lipid-Stoffwechsel störung darstellt.

3. SGLT-2-Inhibitor zur Verwendung in einem Verfahren nach Anspruch 1, wobei die Stoffwechselmyopathie eine Glycogenspeicherkrankheit (GSD), einen Fettsäureoxidationsdefekt (FAODs) oder eine mitochondriale Myopathie darstellt.

4. SGLT-2-Inhibitor zur Verwendung in einem Verfahren nach Anspruch 1, 2 oder 3, wobei die Verabreichung zu einer besseren Toleranz gegenüber Leibesübungen und/oder geringerer Ermüdung und Krämpfen führt.

5. SGLT-2-Inhibitor zur Verwendung in einem Verfahren nach Anspruch 1, 2 oder 3, wobei die Verabreichung Muskelschäden und/oder damit verbundene Rhabdomyolyse und Myoglobinurie reduziert.

6. SGLT-2-Inhibitor zur Verwendung in einem Verfahren nach irgendeinem der Ansprüche 1 bis 5, wobei der Patient ein Patient ist, der Diabetes mellitus Typ 2 hat.

7. SGLT-2-Inhibitor zur Verwendung in einem Verfahren nach irgendeinem der Ansprüche 1 bis 6, wobei der SGLT-2-Inhibitor Empagliflozin darstellt.

8. SGLT-2-Inhibitor zur Verwendung in einem Verfahren nach irgendeinem der Ansprüche 1 bis 7, wobei der Dosisbereich des SGLT-2-Inhibitors 1 bis 50 mg pro Tag beträgt.

9. SGLT-2-Inhibitor zur Verwendung in einem Verfahren nach Anspruch 7, wobei die Dosisstärke des SGLT-2-Inhibitors Empagliflozin 10 mg oder 25 mg einmal am Tag oder 5 mg oder 12,5 mg zweimal am Tag beträgt.

10. SGLT-2-Inhibitor zur Verwendung in einem Verfahren nach irgendeinem der Ansprüche 1 bis 9, wobei die Verabreichung des SGLT-2-Inhibitors an den Patienten zusätzlich zu einer spezifischen Diät erfolgt, die der Patient einnimmt, um die Energieproduktion in den Organen, insbesondere in den Muskeln, zu erhöhen.

## Revendications

1. Inhibiteur de SGLT-2 pour son utilisation dans une méthode de traitement, de retardement ou de ralentissement de la progression d'une myopathie métabolique chez un patient, ladite méthode comprenant l'administration au patient de l'inhibiteur de SGLT-2 dans lequel l'inhibiteur de SGLT-2 est sélectionné parmi un dérivé benzène à substitution glucopyranosyle de formule (I)

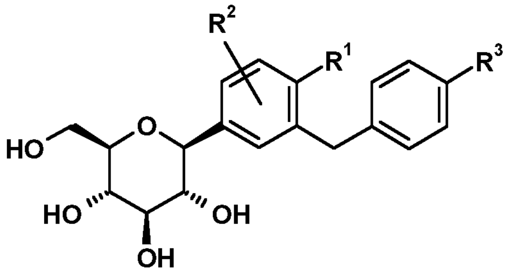

I

dans lequel $R^1$ indique Cl ou un méthyle ; $R^2$ indique H, un méthyle, un méthoxy ou un hydroxy et $R^3$ indique un éthyle, un cyclopropyle, un éthynyle, un éthoxy, un (R)-tétrahydrofuran-3-yloxy ou un (S)-tétrahydrofuran-3-yloxy.

2. Inhibiteur de SGLT-2 pour son utilisation dans une méthode selon la revendication 1, dans lequel ladite myopathie métabolique est une myopathie métabolique parmi un trouble du glycogène ou du métabolisme lipidique.

3. Inhibiteur de SGLT-2 pour son utilisation dans une méthode selon la revendication 1, dans lequel ladite myopathie est une maladie du stockage du glycogène (GSD), un défaut d'oxydation des acides gras (FAOD) ou une myopathie mitochondriale.

4. Inhibiteur de SGLT-2 pour son utilisation dans une méthode selon la revendication 1, 2 ou 3 dans lequel l'administration conduit à une meilleure tolérance à l'exercice et/ou une fatigabilité et des crampes moindres.

5. Inhibiteur de SGLT-2 pour son utilisation dans une méthode selon la revendication 1, 2 ou 3 dans lequel l'administration réduit les lésions musculaires et/ou la rhabdomyolyse et la myoglobinurie associées.

6. Inhibiteur de SGLT-2 pour son utilisation dans une méthode selon l'une quelconque des revendications 1 à 5, dans lequel ledit patient est un patient présentant un diabète sucré de type 2.

7. Inhibiteur de SGLT-2 pour son utilisation dans une méthode selon l'une quelconque des revendications 1 à 6, dans lequel l'inhibiteur de SGLT-2 est l'empagliflozine.

8. Inhibiteur de SGLT-2 pour son utilisation dans une méthode selon l'une quelconque des revendications 1 à 7, dans lequel la plage posologique de l'inhibiteur de SGLT-2 est de 1 à 50 mg/jour.

9. Inhibiteur de SGLT-2 pour son utilisation dans une méthode selon la revendication 7, dans lequel le dosage de l'inhibiteur de SGLT-2 empagliflozine est de 10 mg ou 25 mg une fois par jour ou de 5 mg ou 12,5 mg deux fois par jour.

10. Inhibiteur de SGLT-2 pour son utilisation dans une méthode selon l'une quelconque des revendications 1 à 9, dans lequel ladite administration de l'inhibiteur de SGLT-2 au patient vient compléter un régime alimentaire spécifique pris par le patient pour augmenter la production d'énergie dans les organes, notamment dans les muscles.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0127128 A
- WO 03099836 A
- WO 2005092877 A
- WO 2006034489 A
- WO 2006064033 A
- WO 2006117359 A
- WO 2006117360 A
- WO 2007025943 A
- WO 2007028814 A
- WO 2007031548 A
- WO 2007093610 A
- WO 2007128749 A
- WO 2008049923 A
- WO 2008055870 A
- WO 2008055940 A
- CN 101638423
- WO 2006120208 A
- WO 2010092126 A
- WO 2011039107 A


### Non-patent literature cited in the description


- **FORD ES et al.** *JAMA,* 2002, vol. 287, 356-9
- **KATSUKI A et al.** *Diabetes Care,* 2001, vol. 24, 362-5
- **MATTHEWS et al.** *Diabetologia,* 1985, vol. 28, 412-19
- **FORST et al.** *Diabetes,* 2003, vol. 52 (1), A459
- **GALVIN P et al.** *Diabet Med,* 1992, vol. 9, 921-8
- **STUMVOLL et al.** *Eur J Clin Invest,* 2001, vol. 31, 380-81
- **J. B. MEIGS et al.** *Diabetes,* 2003, vol. 52, 1475-1484
- *Diabetes Care,* 2002, vol. 25, 742-749
- **LAAKSONEN DE et al.** *Am J Epidemiol,* 2002, vol. 156, 1070-7
- *JAMA: Journal of the American Medical Association,* 2001, vol. 285, 2486-2497
- **LAAKSONEN DE et al.** *Am J Epidemiol.,* 2002, vol. 156, 1070-7